# EUROPÄISCHE PATENTANMELDUNG

(11) **EP 1 864 909 A1**
(43) Veröffentlichungstag der Anmeldung: **12.12.2007**
(21) Anmeldenummer: 07010960.8
(22) Anmeldetag: 04.06.2007
(51) Int. Cl.: B65B 55/10, A61L 2/14, B65D 77/00

(54) **Sterilisierte Cyanoacrylatverpackung**

(30) Priorität: 06.06.2006 DE 102006027305
(71) Anmelder: Aesculap AG & Co. KG, 78532 Tuttlingen/Donau (DE)
(72) Erfinder: Siedle, Gabriel, 79117 Freiburg (DE); Odermatt, Erich, 8200 Schaffhausen (CH); Wegmann, Jürgen, 78333 Stockach (DE)
(74) Vertreter: Patentanwälte Ruff, Wilhelm, Beier, Dauster & Partner

(57) **Zusammenfassung**

Die Erfindung betrifft eine sterilisierte Verpackung (30) mit einem inneren im Wesentlichen gasdichten und geschlossenen Verpackungsmittel (31), das eine sterile und zu eine zu einem Kleber aushärtbare Klebstoffzusammensetzung (32) enthält; und einem äußeren Verpackungsmittel (33), das das innere Verpackungsmittel (31) umgibt, wobei die Verpackung (1; 10; 20; 30) zwischen dem inneren Verpackungsmittel (31) und dem äußeren Verpackungsmittel (33) ein steriles Verpackungsvolumen (35) aufweist. Die Erfindung betrifft weiterhin ein Verfahren zur Herstellung der Verpackung (30) sowie ihre Verwendung zur sterilen Entnahme des inneren Verpackungsmittels (31) aus der Verpackung (30).

## Beschreibung

Die Erfindung betrifft eine sterilisierte Verpackung, ein diesbezügliches Sterilisationsverfahren sowie eine Verwendung der Verpackung.

Für die Sterilisation von Klebstoffzusammensetzungen stehen heutzutage eine Vielzahl verschiedener Sterilisationsverfahren zur Verfügung. Zu den am häufigsten angewandten Verfahren zählen insbesondere die Gammabestrahlung und Elektronenstrahl-Bestrahlung. Nachteilig hierbei ist jedoch eine häufig auftretende Polymerisation der zu sterilisierenden Klebstoffzusammensetzungen, insbesondere auf der Basis von Cyanoacrylat-Monomeren. Allerdings lassen sich derartige Klebstoffzusammensetzungen unter bestimmten Voraussetzungen insbesondere durch Gamma-oder Elektronenstrahl-Bestrahlung sterilisieren. Ein derartiges Sterilisationsverfahren ist insbesondere aus der DE 20 55 658 B1 bekannt, wonach Klebstoffzusammensetzungen auf der Basis von Cyanoacrylat-Monomeren bei einer Sterilisationstemperatur von ungefähr - 30°C sowie durch Zugabe von Stabilisatoren ohne Aushärtung der Klebstoffzusammensetzungen sterilisiert werden können. Weitere Beispiele für Sterilisationsverfahren auf der Basis von Gamma- oder Elektronenstrahlbestrahlung können der EP 1 056 480 B1, EP 1 206 291 sowie der EP 0 659 441 B entnommen werden.

Klebstoffzusammensetzungen, welche im Wesentlichen auf Cyanoacrylat-Monomeren beruhen, zählen zu den wichtigsten synthetischen Klebstoffzusammensetzungen in der modernen Klebechirurgie. Ein Beispiel für eine Klebstoffzusammensetzung auf Basis von n-Butyl-Cyanoacrylat wird von der B. Braun Melsungen AG kommerziell unter der Bezeichnung Histoacryl^{®} vertrieben. Zur Aufbewahrung bzw. Lagerung von sterilen Klebstoffzusammensetzungen werden insbesondere Glas- oder Kunststoffampullen verwendet, welche wiederum häufig in verpackter Form, beispielsweise in Form von Verpackungsbeuteln oder Tiefziehverpackungen, vorliegen. Nachteilig ist, dass in vielen Fällen eine Sterilisierung der verpackten Ampullen wegen einer vorzeitigen Aushärtung oder Zersetzung der Klebstoffzusammensetzungen in den Ampullen infolge der Sterilisierung nicht in Frage kommt. Demgegenüber bestehen jedoch gerade in der modernen Versorgungsmedizin erhöhte Anforderungen an die Sterilität zur Verbesserung der Sicherheitsstandards in medizinischen Behandlungsräumen.

Somit stellt sich die Erfindung die Aufgabe, eine sterilisierte Verpackung sowie ein entsprechendes Sterilisationsverfahren bereitzustellen, welche jeweils den erhöhten Anforderungen an die Sterilität gerecht werden.

Die Aufgabe wird gelöst durch eine sterilisierte Verpackung mit einem inneren im Wesentlichen gasdichten und geschlossenen Verpackungsmittel, das eine sterile und zu einem Kleber aushärtbare Klebstoffzusammensetzung enthält, und einem äußeren Verpackungsmittel, das das innere Verpackungsmittel umgibt, wobei die Verpackung zwischen dem inneren und äußeren Verpackungsmittel ein steriles und trockenes Verpackungsvolumen aufweist.

Durch die Erfindung werden sterilisierte Verpackungen mit einem inneren und äußeren Verpackungsmittel bereitgestellt, wobei das innere Verpackungsmittel eine sterile und vorzugsweise trockene Außenseite besitzt. Zudem enthält das innere Verpackungsmittel eine zu einem Kleber aushärtbare Klebstoffzusammensetzung, welche trotz der Sterilität der Verpackung hohen bis sehr hohen Qualitätsanforderungen entspricht. Die gemäß der Erfindung bereitgestellten Verpackungen eignen sich insbesondere für eine sterile Entnahme des inneren Verpackungsmittels (mit der darin befindlichen Klebstoffzusammensetzung) aus der Verpackung. Dies ist in besonderem Maße in der Medizin, insbesondere der Chirurgie, erwünscht. Somit leistet die Erfindung einen wertvollen Beitrag zur Erhöhung der Sterilisationsstandards und damit der allgemeinen Sicherheit vor möglichen Infektionen in medizinischen Behandlungsräumen, insbesondere in OP-Bereichen (Operationsbereichen).

Unter einer aushärtbaren Klebstoffzusammensetzung im Sinne der vorliegenden Erfindung soll eine Klebstoffzusammensetzung verstanden werden, welche außerhalb des inneren Verpackungsmittels, insbesondere bei Raumtemperatur, vorzugsweise bei Körpertemperatur von Mensch und/oder Tier, aushärtbar ist.

In einer bevorzugten Ausführungsform weist das äußere Verpackungsmittel eine Sterilisationsöffnung, insbesondere eine gasdurchlässige und keimdichte Sterilisationsöffnung, auf. Bei der insbesondere gasdurchlässigen und keimdichten Sterilisationsöffnung handelt es sich vorzugsweise um eine Wandung, insbesondere um einen Wandungsteil, des äußeren Verpackungsmittels. Erfindungsgemäß ist es insbesondere vorgesehen, dass es sich bei der Sterilisationsöffnung um ein sogenanntes Sterilisationsfenster handelt, das insbesondere einseitig mit dem äußeren Verpackungsmittel integriert ist.
Mit besonderem Vorteil ist das äußere Verpackungsmittel vollständig aus einem gasdurchlässigen und keimdichten Material gebildet.

Das äußere Verpackungsmittel der erfindungsgemäßen Verpackung weist vorzugsweise eine Sterilisationsöffnung, insbesondere eine gasdurchlässige und keimdichte Sterilisationsöffnung, auf, die gasdicht verschlossen ist. Bevorzugt ist die Sterilisationsöffnung mit einer gasdichten Folie, insbesondere einer gasdichten Abdeckfolie verschlossen. Die Folie kann insbesondere aus Metallen, vorzugsweise aus Aluminium, oder Verbundmaterialien gebildet sein. Weiterhin kann die Sterilisationsöffnung mit Hilfe von Schweiß- oder Klebetechniken verschlossen sein. Bezüglich weiterer Einzelheiten wird vollumfänglich auf die vorliegende Beschreibung Bezug genommen.

In einer weitergehenden Ausführungsform weist das äußere Verpackungsmittel eine Sterilisationsöffnung aus einem Faservliesmaterial, insbesondere aus einem polymeren Faservliesmaterial, auf. Besonders bevorzugt ist das Faservliesmaterial aus Polyethylen, vorzugsweise aus Hochdruckpolyethylen, gebildet. Bei dem Faservliesmaterial kann es sich insbesondere um das unter der Bezeichnung Tyvek® kommerziell erhältliche Produkt handeln.

Weiterhin kann das Faservliesmaterial aus Polypropylen, insbesondere aus Hochdruckpolypropylen, gebildet sein. Beispielsweise ist ein derartiges Faservliesmaterial unter der Bezeichnung Typar^{®} kommerziell erhältlich.

Die Verpackungsmittel der erfindungsgemäßen Verpackung können in unterschiedlichen dreidimensionalen Formen, insbesondere als Beutel, Röhrchen oder ähnliches, vorliegen. Bevorzugt liegen die Verpackungsmittel, insbesondere das äußere Verpackungsmittel, als Tiefziehverpackungen vor.

Bei dem inneren Verpackungsmittel der erfindungsgemäßen Verpackung kann es sich insbesondere um Ampullen, einen Spritzenzylinder, insbesondere Einkammer- oder Zweikammerspritzen, oder Tubes handeln. Das innere Verpackungsmittel kann insbesondere schichtförmig aufgebaut sein und beispielsweise als Mehrschichtampulle vorliegen. Das innere Verpackungsmittel ist vorzugsweise aus Kunstoffen, Glas und/oder Metallen gebildet. Bei den Kunststoffen handelt es sich vorzugsweise um Copolymere, insbesondere auf der Basis von Ethylen und/oder Propylen. Bevorzugt handelt es sich bei den Kunststoffen um Cycloolefin-Copolymere, insbesondere auf der Basis von Norbornen und Ethylen. Ein besonders bevorzugter Kunststoff ist unter der Bezeichnung TOPAS^{®} kommerziell erhältlich. Weiterhin ist es erfindungsgemäß insbesondere vorgesehen, dass das innere Verpackungsmittel aus sogenannten Verbundmaterialien gebildet ist. Die Verbundmaterialien können mit besonderem Vorteil mit einer Wasserdampfsperrschicht, beispielsweise aus Siloxanen oder Polyvinylidenchlorid (PVDC), versehen sein. Mit Vorteil ist das innere Verpackungsmittel eine Folie, die vorzugsweise dampfdicht, insbesondere wasserdampfdicht, ausgebildet ist.

In einer besonders bevorzugten Ausführungsform ist das innere Verpackungsmittel eine Ampulle, insbesondere auf der Basis von Kunststoffen. Vorzugsweise handelt es sich bei dem inneren Verpackungsmittel um eine reine Kunststoffampulle.

In einer weiteren insbesondere bevorzugten Ausführungsform ist das äußere Verpackungsmittel aus gasdichten, insbesondere dampfdichten, Materialien, vorzugsweise aus Metallen oder Verbundmaterialien, gebildet. Vorzugsweise ist das Verpackungsvolumen bzw. der Innenraum des äußeren Verpackungsmittels frei von Feuchtigkeit. Bei den Metallen handelt es sich bevorzugt um Aluminium. Die Verbundmaterialien sind insbesondere schichtförmig aufgebaut und können beispielsweise Siegel- bzw. Klebeschichten aufweisen. Mit Vorteil handelt es sich bei den Verbundmaterialien um Laminate. Besonders bevorzugt ist das äußere Verpackungsmittel der erfindungsgemäßen Verpackung aus Verbundmaterialien auf der Basis von Metallen, Papier, Kunstoffen oder Lacken gebildet. Die Verbundmaterialien können insbesondere eine der im Folgendenden aufgeführten Zusammensetzungen aufweisen:
Polyethylen/Aluminium/Polyethylenterephthalat, Polyvinylchlorid/Aluminium/Polyamid, Polypropylen/Aluminium/Lacke, Polyethylenterephthalat/Aluminium/Polypropylen und Lacke/Aluminium/Polyethylenterephthalat/Papier.

In einer bevorzugten Ausführungsform ist der SAL (sterile assurance level) zwischen dem inneren und äußeren Verpackungsmittel der erfindungsgemäßen Verpackung < 10⁻³, vorzugsweise ≤ 10⁻⁶.

In einer weitergehenden Ausführungsform sind das innere und/oder äu-βere Verpackungsmittel, vorzugsweise das innere und äußere Verpackungsmittel, frei von Sterilisationsrückständen. Vorzugsweise sind das innere und/oder das äußere Verpackungsmittel, vorzugsweise das innere und äußere Verpackungsmittel, frei von Sterilisationsgasen. Auf diese Weise wird insbesondere eine negative Beeinflussung der Klebeeigenschaften der Klebstoffzusammensetzung durch die Sterilisationsrückstände vermieden.

In einer weitergehenden bevorzugten Ausführungsform weist die sterile Klebstoffzusammensetzung in dem inneren Verpackungsmittel während eines Zeitraums von 2 bis 60 Monaten, insbesondere von 4 bis 36 Monaten, vorzugsweise von 6 bis 24 Monaten, nach Sterilisation der Verpackung eine Viskosität auf, die im Wesentlichen der Viskosität der sterilen Klebstoffzusammensetzung in dem inneren Verpackungsmittel vor Sterilisation der Verpackung entspricht.
Mit besonderem Vorteil weist die sterile Klebstoffzusammensetzung in dem inneren Verpackungsmittel während eines Zeitraums von 2 bis 60 Monaten, insbesondere von 4 bis 36 Monaten, vorzugsweise von 6 bis 24 Monaten, nach Sterilisation der Verpackung einen Wassergehalt auf, die im Wesentlichen dem Wassergehalt der sterilen Klebstoffzusammensetzung in dem inneren Verpackungsmittel vor Sterilisation der Verpackung entspricht.

In einer insbesondere bevorzugten Ausführungsform weist die sterile Klebstoffzusammensetzung in dem inneren Verpackungsmittel während eines Zeitraums von mindestens 2 Monaten, insbesondere von mindestens 4 Monaten, vorzugsweise von mindestens 6 Monaten, nach Sterilisation der Verpackung eine Viskosität auf, die im Wesentlichen der Viskosität der sterilen Klebstoffzusammensetzung in dem inneren Verpackungsmittel vor Sterilisation der Verpackung entspricht.

In einer weiteren Ausführungsform weist die sterile Klebstoffzusammensetzung in dem inneren Verpackungsmittel während eines Zeitraums von mindestens zwei Monaten, insbesondere von mindestens vier Monaten, vorzugsweise von mindestens sechs Monaten, nach Sterilisation der Verpackung eine Haftkraft auf, die im Wesentlichen der Haftkraft der sterilen Klebstoffzusammensetzung in dem inneren Verpackungsmittel vor Sterilisation der Verpackung entspricht.

In einer bevorzugten Ausführungsform der erfindungsgemäßen Verpackung weist die Klebstoffzusammensetzung in dem inneren Verpackungsmittel Cyanoacrylat-Monomere auf. Die Cyanoacrylat-Monomere können insbesondere als Mischung von verschiedenen Cyanaocrylat-Monomeren vorliegen. Bevorzugt weist die Klebstoffzusammensetzung Alkyl-Cyanoacrylat-Monomere und/oder Alkoxy-Alkyl-Cyanoacrylat-Monomere und/oder Alkylester-Cyanoacrylat-Monomere auf. Die Alkylketten der Cyanoacrylat-Monomere weisen insbesondere eine Kohlenstoffanzahl zwischen 1 bis 12, insbesondere zwischen 1 und 10, auf. Erfindungsgemäß ist es besonders bevorzugt, dass es sich bei den Alkyl-Cyanoacrylat-Monomeren um mindestens ein Monomer aus der Gruppe, umfassend Ethyl-, n-Butyl-, iso-Butyl- und n-Octyl-Cyanoacrylat, handelt.
Bei den Alkoxy-Alkyl-Cyanoacrylat-Monomeren handelt es sich bevorzugt um Methoxypropyl- und/oder Ethoxyethyl-Cyanoacrylat, vorzugsweise um Methoxypropyl-Cyanoacrylat.
Die Alkylester-Cyanoacrylat-Monomere sind bevorzugt aus der Gruppe, umfassend Butyllactoyl-, Butylglykoloyl-, lsopropylglykoloyl-, Ethyllactoyl- und Ethylglykoloyl-Cyanoacrylat, ausgewählt.

Erfindungsgemäß ist es weiterhin vorgesehen, dass die Klebstoffzusammensetzung zusätzliche Additive aufweist. Bei den Additiven kann es sich insbesondere um Weichmacher handeln. Als Weichmacher kommen insbesondere alle dem Fachmann geläufigen Stoffe in Frage. Weiterhin kann die Klebstoffzusammensetzung Verbindungen aufweisen, welche die Viskosität der Klebstoffzusammensetzung beeinflussen. Bevorzugt handelt es sich bei diesen Stoffen um Polymere, vorzugsweise um resorbierbare Polymere. Erfindungsgemäß ist es insbesondere vorgesehen, dass die Polymere als Co- oder Terpolymere vorliegen, vorzugsweise auf der Basis von mindestens einem Monomer aus der Gruppe Lactid, Glykolid, Caprolacton, Trimethylencarbonat, p-Dioxanon und Hydroxybuttersäure. Besonders bevorzugt weist die Klebstoffzusammensetzung ein Copolymer aus Lactid und Caprolacton auf. Weiterhin kann die Klebstoffzusammensetzung ein Terpolymer auf der Basis von Trimethylencarbonat, Glykolid und Caprolacton aufweisen. Erfindungsgemäß kann es ebenso von Vorteil sein, dass die Klebstoffzusammensetzung Poly-Dioxanon aufweist.

In einer weiteren insbesondere bevorzugten Ausführungsform weist die Klebstoffzusammensetzung Stabilisatoren, insbesondere zur Verhinderung einer vorzeitigen Polymerisation der in der Klebstoffzusammensetzung vorhandenen aushärtbaren Komponenten, auf. Bevorzugt handelt es sich bei den Stabilisatoren um mindestens einen Stabilisator aus der Gruppe Schwefeldioxid, Phosphorsäure, Hydrochinon, tert.-ButylHydroxyanisol, Essigsäure und Schwefelsäure. Die einzelnen Stabilisatoren der Klebstoffzusammensetzung können in unterschiedlichen Mengen vorhanden sein. Erfindungsgemäß liegen die Stabilisatoren bevorzugt in den folgenden Mengenbereichen vor: Schwefeldioxid: 20 bis 100 ppm, Phosphorsäure: 10 bis 500 ppm, Hydrochinon: 100 bis 3000 ppm, tert.-Butyl-Hydroxyanisol: 100 bis 3000 ppm, Essigsäure: 10 bis 500 ppm und Schwefelsäure: 10 bis 500 ppm.

Die Erfindung betrifft weiterhin ein Verfahren zur Sterilisation einer gasdurchlässigen und keimdichten Verpackung mit einem inneren im Wesentlichen gasdichten und geschlossenen Verpackungsmittel, welches eine sterile und zu einem Kleber aushärtbare Klebstoffzusammensetzung enthält, und einem äußeren Verpackungsmittel, welches das innere Verpackungsmittel umgibt, wobei die Verpackung einer Gassterilisation unter Erhalt der Aushärtbarkeit der Klebstoffzusammensetzung und unter Ausbildung eines sterilen und trockenen Verpackungsvolumens zwischen dem inneren und äußeren Verpackungsmittel unterworfen wird.

In einer insbesondere bevorzugten Ausführungsform wird die Verpackung vor der Gassterilisation in ein gasdurchlässiges und keimdichtes Behältnis eingeschlossen. Als Behältnis kann insbesondere ein Tyvek®-Beutel oder ein Aluminiumbeutel mit einem Sterilisationsfenster, insbesondere mit einem Tyvek®-Fenster, verwendet werden. Bezüglich weiterer Einzelheiten wird auf die vorliegende Beschreibung verwiesen.

In einer besonders bevorzugten Ausführungsform wird nach der Gassterilisation eine Sterilisationsöffnung, insbesondere eine gasdurchlässige und keimdichte Sterilisationsöffnung, vorzugsweise in Form eines Wandungsteils, des äußeren Verpackungsmittels gasdicht verschlossen. Bezüglich weiterer Einzelheiten wird auf die bisherige Beschreibung Bezug genommen. Vor dem gasdichten Verschließen wird der Innenraum des äußeren Verpackungsmittels vorzugsweise getrocknet, d.h. von jeglicher Restfeuchtigkeit befreit.

In einer bevorzugten Ausführungsform wird die Verpackung in Gegenwart von gasförmigen Radikalen, insbesondere von Sauerstoffradikalen, sterilisiert. Besonders bevorzugt wird die Verpackung mit von Wasserstoffperoxid abgeleiteten Radikalen sterilisiert. Dies ist besonders vorteilhaft, da es sich bei Radikalen um äußerst aggressive chemische Spezies handelt, die in vorteilhafter Weise eine schnelle Sterilisierung des zu sterilisierenden Gegenstandes bewirken. Es wird eine Verpackung mit sterilem Inhalt erhalten. Durch die Verwendung eines inneren Verpackungsmittels, das im Wesentlichen undurchlässig für Gase und somit insbesondere auch für gasförmige Radikale undurchlässig ist, werden bei dem erfindungsgemäßen Verfahren unerwünschte Reaktionen zwischen der sterilen Klebstoffzusammensetzung und den Radikalen vermieden.

In einer weiteren Ausführungsform wird die Verpackung in Gegenwart eines mindestens teilweise ionisierten Gases sterilisiert.

In einer weitergehenden besonders bevorzugten Ausführungsform wird die Verpackung einer sogenannten Plasma-Sterilisation unterworfen. Überraschender Weise wurde festgestellt, dass bei Verwendung von inneren Verpackungsmitteln aus Kunststoff eine Plasma-Sterilisation der Verpackung ohne Beeinträchtigung der Qualität der in den inneren Verpackungsmitteln befindlichen sterilen Klebstoffzusammensetzungen durchgeführt werden kann. Dies ist besonders vorteilhaft, da gerade Verpackungsmittel aus Kunststoffen, insbesondere Kunststoffampullen, aufgrund ihrer kostengünstigen Herstellung und einfachen Handhabung die häufigsten Verpackungsformen für Klebstoffzusammensetzungen in der Medizin darstellen.

Unter einem Plasma wird physikalisch ein mindestens teilweise ionisiertes Gas mit einem bestimmten Anteil an freien Ladungsträgern verstanden. Bei den Ladungsträgern handelt es sich insbesondere um Ionen, Elektronen und Radikale. Das Plasma kann ein Niederdruck- oder Normaldruckplasma sein. Erfindungsgemäß ist es bevorzugt, die Sterilisation mit einem Niederdruckplasma vorzunehmen. Die Herstellung des Plasmas wird mit Vorteil durch Niederdruckentladungen durchgeführt, insbesondere durch eine Hochfrequenz, Mikro- oder Radiowellen. Die sterilisierende Wirkung des Plasmas ist hauptsächlich auf die Bildung chemisch aggressiver Substanzen, insbesondere von Radikalen, sowie auf den Beschuß von Keimen, d.h. Mikroorganismen und Viren, mit lonen zurückzuführen.

In einer erfindungsgemäß ganz besonders bevorzugten Ausführungsform wird die Verpackung einer Wasserstoffperoxid-Plasma-Sterilisation unterworfen. Zur Herstellung des Wasserstoffperoxid-Plasmas wird vorzugsweise eine Wasserstoffperoxid-Lösung in einen sterilen Raum verdunstet. Bevorzugt wird eine Wasserstoffperoxid-Lösung mit einem Anteil von ca. 58 Gew.-% Wasserstoffperoxid, bezogen auf das Gesamtgewicht der Lösung, verwendet. Der infolge der Verdunstung entstandene Dampf von Wasserstoffperoxid kann insbesondere durch Einwirkung von Radiofrequenzenergie in ein Plasma umgewandelt werden.

Bei der Plasma-Sterilisation werden grundsätzlich keine giftigen Rückstände und/oder Emissionen gebildet. Außerdem ist der Energieeintrag in die sterile Klebstoffzusammensetzung bei der Plasma-Sterilisation während des Sterilisationsvorganges vernachlässigbar gering, so dass ein Qualitätsverlust der sterilen Klebstoffzusammensetzung, insbesondere durch Zersetzung oder vorzeitige Aushärtung, nicht eintritt.

In einer anderen Ausführungsform des erfindungsgemäßen Verfahrens wird die Verpackung mit Wasserstoffperoxid-Gas sterilisiert, wobei die Sterilisation mit Wasserstoffperoxid-Dampf bevorzugt ist. Mit besonderem Vorteil wird für die Herstellung von Wasserstoffperoxid-Dampf eine Wasserstoffperoxid-Lösung mit einem Anteil von ca. 35 Gew.-% Wasserstoffperoxid, bezogen auf das Gesamtgewicht der Lösung, verwendet. In einer wiederum anderen Ausführungsform des erfindungsgemäßen Verfahrens wird die Verpackung mit Formaldehyd-Gas, vorzugsweise mit Formaldehyd-Dampf, sterilisiert. Mit besonderem Vorteil wird für die Herstellung von Formaldehyd-Dampf eine Formaldehyd-Lösung mit einem Anteil von 2 Gew.-% Formaldehyd, bezogen auf das Gesamtgewicht der Lösung, verwendet. Sowohl Wasserstoffperoxid als auch Formaldehyd stellen antimikrobiell äußerst wirksame Verbindungen dar und eignen sich im Rahmen des erfindungsgemäßen Verfahrens zur Sterilisierung der Verpackung.

In einer bevorzugten Ausführungsform des erfindungsgemäßen Verfahrens wird die Verpackung bei Unterdruck sterilisiert. Vorzugsweise wird die Verpackung bei einem Unterdruck zwischen 0,005 und 200 Torr, insbesondere zwischen 0,01 und 100 Torr, vorzugsweise zwischen 0,1 und 50 Torr, sterilisiert. Besonders bevorzugt wird die Sterilisierung der Verpackung mittels einer Plasma-Sterilisation bei Unterdruck (Niederdruckplasma-Sterilisation) durchgeführt.

In einer anderen Ausführungsform des erfindungsgemäßen Verfahrens wird die Verpackung bei Überdruck sterilisiert. Bevorzugt wird die Verpackung bei einem Überdruck zwischen 1 und 5 bar, vorzugsweise zwischen 1,05 und 5 bar, sterilisiert.

Die Sterilisation mit Wasserstoffperoxid wird insbesondere bei Unterdruck durchgeführt. Weiterhin wird die Sterilisation mit Formaldehyd-Gas mit Vorteil bei Unterdruck, vorzugsweise bei einem Unterdruck zwischen 20 und 400 mbar, insbesondere zwischen 50 und 200 mbar, durchgeführt.

In einer weiteren bevorzugten Ausführungsform des erfindungsgemäßen Verfahrens wird die Verpackung bei einer Temperatur zwischen 20°C und 75°C, insbesondere bei einer Temperatur zwischen 25°C und 60°C, vorzugsweise bei einer Temperatur zwischen 30°C und 50°C, sterilisiert. Abhängig von der Art der Gassterilisation können engere Temperaturbereiche bevorzugt sein. Erfindungsgemäß ist es besonders bevorzugt, dass bei der Sterilisierung der Verpackung durch Plasma, vorzugsweise durch Wasserstoffperoxid-Plasma, bei einer Temperatur von ca. 45°C gearbeitet wird. Die Sterilisierung der Verpackung mit Wasserstoffperoxid-Gas wird vorteilhafter Weise in einem Temperaturbereich von 30°C bis 40°C durchgeführt. Die Sterilisierung der Verpackung mit Formaldehyd-Gas wird mit besonderem Vorteil in einem Temperaturbereich zwischen 50 °C und 75 °C, vorzugsweise bei einer Temperatur von ca. 50 °C, ca. 60°C oder ca. 75 °C, vorgenommen. Somit kann die Sterilisierung der Verpackung hinsichtlich der Sterilisationstemperaturen abhängig von der Art der Gassterilisation unter schonenden bis äußerst schonenden Bedingungen durchgeführt werden. Dies ist einerseits vorteilhaft, da somit ein Wärmeeintrag in die in dem inneren Verpackungsmittel vorliegende sterile Klebstoffzusammensetzung vermieden wird. Andererseits können zur Durchführung des erfindungsgemäßen Verfahrens auch weniger hitzebeständige Materialien für das innere Verpackungsmittel, insbesondere Kunststoffe, verwendet werden. Dies ist in besonderem Maße vorteilhaft, da Verpackungsmittel aus Kunstoffen, wie bereits erwähnt, ohne größeren technischen Aufwand hergestellt werden können und insbesondere einfach in der Handhabung sind.

In einer weiteren insbesondere bevorzugten Ausführungsform des erfindungsgemäßen Verfahrens wird die Verpackung während eines Zeitraums von 30 bis 240 Minuten, insbesondere von 40 bis 120 Minuten, vorzugsweise von ca. 60 Minuten sterilisiert. Erfindungsgemäß ist es besonders bevorzugt, dass die Plasma-Sterilisation, vorzugsweise Wasserstoffperoxid-Plasma-Sterilisation, der Verpackung während eines Zeitraumes von ca. 60 Minuten durchgeführt wird. Dies ist besonders vorteilhaft, da eine kurze Sterilisationsdauer zusätzlich zu einer Verringerung eines möglichen Energieeintrags in das innere Verpackungsmittel mit der sterilen Klebstoffzusammensetzung beiträgt. Auf diese Weise wird mit besonderem Vorteil ein Qualitätsverlust der Klebstoffzusammensetzung, insbesondere durch vorzeitige Aushärtung oder Zersetzung, vermieden.

In einer besonders bevorzugten Ausführungsform des erfindungsgemäßen Verfahrens wird die Klebstoffzusammensetzung spätestens bei Überführung in das innere Verpackungsmittel steril gemacht. Bevorzugt wird die Klebstoffzusammensetzung vor der Überführung in das innere Verpackungsmittel steril gemacht. Vorzugsweise wird die Klebstoffzusammensetzung durch Sterilfiltration und/oder aseptische Abfüllung, vorzugsweise durch Sterilfiltration und aseptische Abfüllung, steril gemacht. Mit besonderem Vorteil werden für die Sterilfiltration Sterilfilter mit einer Porengröße und -struktur eingesetzt, welche eine vorzeitige Aushärtung der Klebstoffzusammensetzung vermeiden. Bevorzugt werden für die Sterilfiltration Sterilfilter mit einer Porengröße von ca. 0,2 µm verwendet. Weiterhin kann das innere Verpackungsmittel vor seiner Beschickung mit der Klebstoffzusammensetzung einer Desinfektion oder Sterilisierung unterzogen werden. In anderen Fällen kann es sinnvoll sein, die Klebstoffzusammensetzung nach Überführung in das innere Verpackungsmittel steril zu machen, vorzugsweise nach Verschluß des inneren Verpackungsmittels.

Die vorliegende Erfindung umfaßt auch eine Verpackung, welche nach einem der erfindungsgemäßen Verfahren hergestellt bzw. herstellbar ist.

Die Erfindung betrifft außerdem die Verwendung der erfindungsgemäßen Verpackung zur sterilen und trockenen Aufbewahrung des inneren Verpackungsmittels in der Verpackung und zur sterilen Entnahme des inneren Verpackungsmittels aus der Verpackung, insbesondere zur sterilen Entnahme der aushärtbaren Klebstoffzusammensetzung aus dem inneren Verpackungsmittel.

Durch die vorliegende Erfindung können mit besonderem Vorteil sterilisierte Verpackungen bereitgestellt werden, welche jeweils ein inneres Verpackungsmittel mit einer sterilen Außenseite und einer intakten, d.h. aushärtbaren, Klebstoffzusammensetzung aufweisen. Die Verhinderung eines möglichen Qualitätsverlustes der Klebstoffzusammensetzung wird erfindungsgemäß durch die besondere Wahl der Sterilisationsmethoden und insbesondere durch die schonenden Sterilisationsbedingungen gewährleistet. Dies ist, wie bereits mehrfach erwähnt, besonders vorteilhaft für eine sterile Entnahme des inneren Verpackungsmittels aus der Verpackung und trägt allgemein zu verbesserten Sterilisationsstandards im medizinischen, insbesondere chirurgischen, Bereich bei.

Weitere Einzelheiten und Merkmale der vorliegenden Erfindung ergeben sich aus der nachfolgenden Beschreibung bevorzugter Ausführungsformen in Kombination mit den Figuren, Beispielen und Unteransprüchen. In diesen Ausführungsformen können einzelne Merkmale der Erfindung allein oder in Kombination mit anderen Merkmalen verwirklicht sein. Sämtliche Figuren werden hiermit durch ausdrückliche Bezugnahme zum Inhalt dieser Beschreibung gemacht.

In den Figuren ist Folgendes gezeigt:

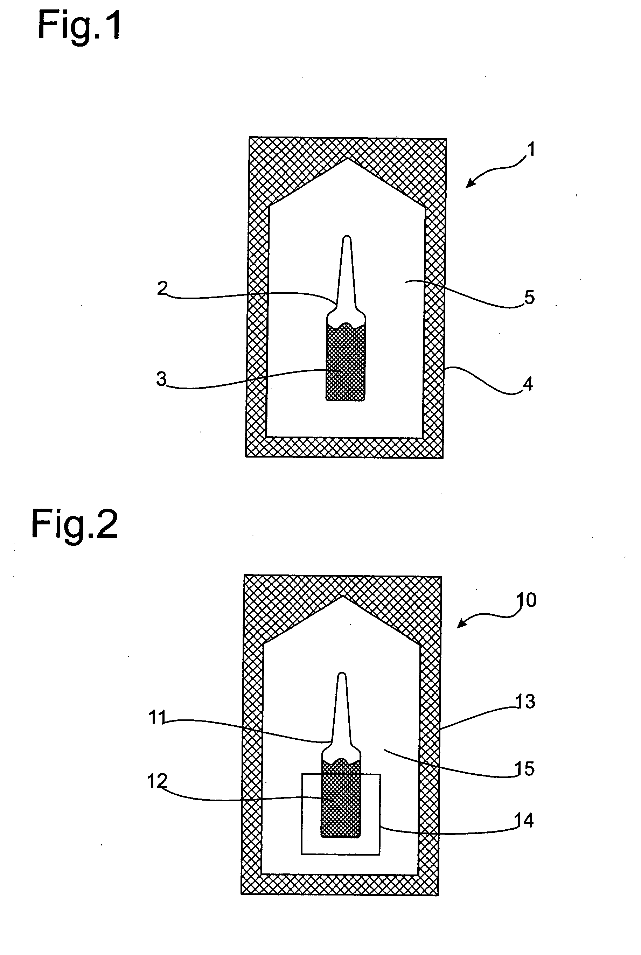

- Fig. 1: Verpackung (1) aus einer als inneres Verpackungsmittel ausgebildeten Kunststoffampulle (2) mit einer sterilen und aushärtbaren Klebstoffzusammensetzung (3) und einem als äußeres Verpackungsmittel ausgebildeten Tyvek®-Beutel (4),
- Fig. 2: Verpackung (10) aus einer als inneres Verpackungsmittel ausgebildeten Kunststoffampulle (11) mit einer sterilen und aushärtbaren Klebstoffzusammensetzung (12) und einem als äußeres Verpackungsmittel ausgebildeten Aluminiumbeutel (13) mit einer Sterilisationsöffnung in Form eines Tyvek®-Fensters (14),

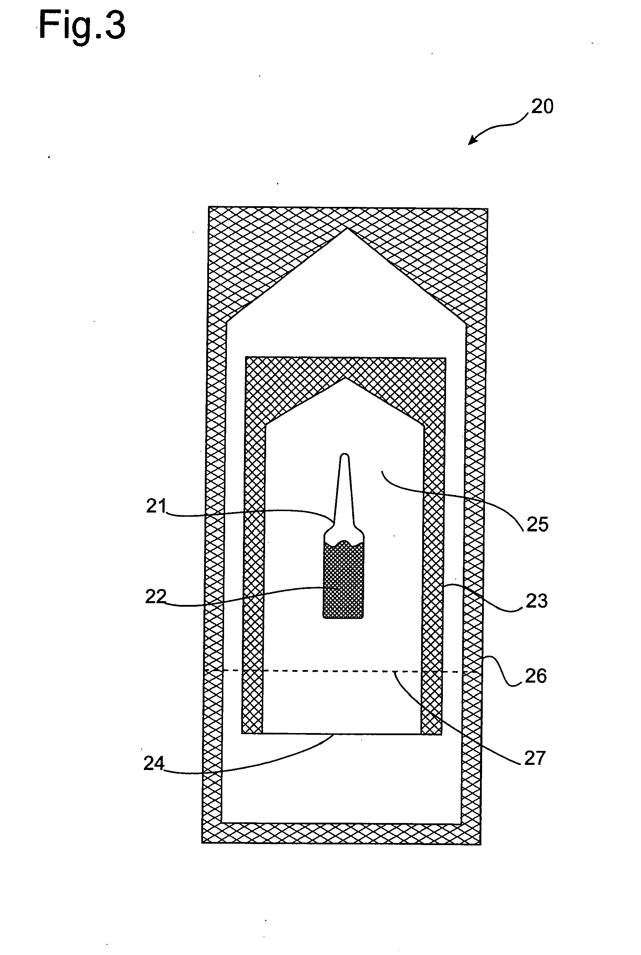

- Fig. 3: Verpackung (20) aus einer als inneres Verpackungsmittel ausgebildeten Kunststoffampulle (21) mit einer sterilen und aushärtbaren Klebstoffzusammensetzung (22) und einem als äußeres Verpackungsmittel ausgebildeten einseitig geöffneten Aluminiumbeutel (23), der in einem Tyvek®-Beutel (26) eingeschlossen ist.

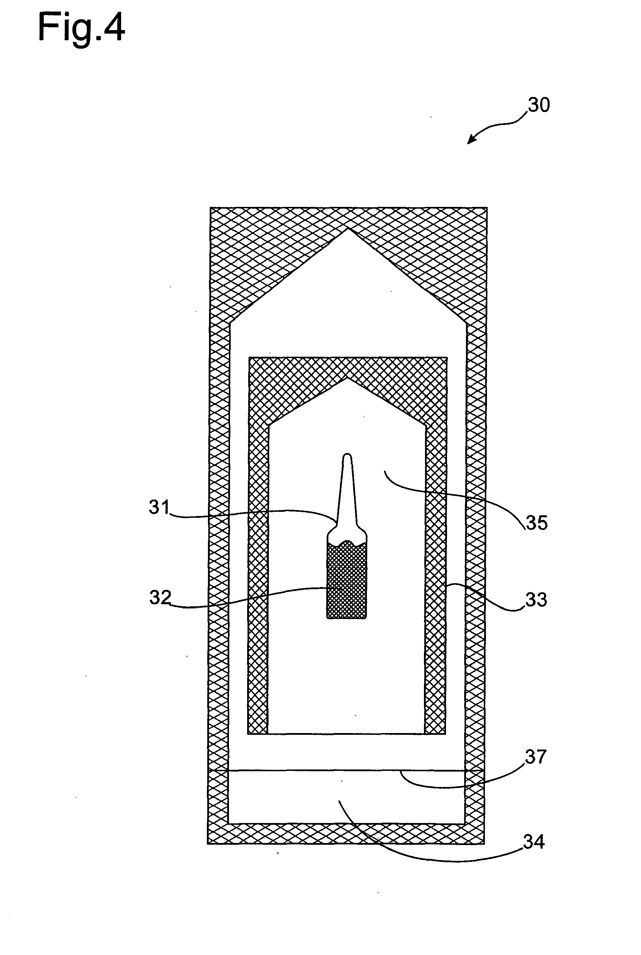

- Fig. 4: Verpackung (30) aus einer als inneres Verpackungsmittel ausgebildeten Kunststoffampulle (31) mit einer sterilen und aushärtbaren Klebstoffzusammensetzung (32) und einem als äußeres Verpackungsmittel ausgebildeten Aluminiumbeutel (33) mit einer Sterilisationsöffnung in Form eines einseitig integrierten Tyvek®-Fensters (34).

### Figurenbeschreibung

Fig. 1 zeigt eine Verpackung (1) aus einer als inneres Verpackungsmittel ausgebildeten Kunststoffampulle (2) mit einer darin befindlichen sterilen und aushärtbaren Klebstoffzusammensetzung und einem als äußeres Verpackungsmittel ausgebildeten Tyvek^{®}-Beutel (4). Der Tyvek^{®}-Beutel (4) umgibt die Kunstoffampulle (2) vollständig. Bei der Klebstoffzusammensetzung (3) innerhalb der Kunststoffampulle (2) handelt es sich um eine Klebstoffzusammensetzung auf der Basis von n-Butyl-Cyanoacrylat (Histoacryl®). Nach Sterilisation der Verpackung (1) ist das Verpackungsvolumen (5) der Verpackung (1) steril.
Fig. 2 zeigt eine Verpackung (10) mit einer als inneres Verpackungsmittel ausgebildeten Kunststoffampulle (11), die eine sterile und aushärtbare Klebstoffzusammensetzung (12) aus einem Gemisch aus n-Butyl- und n-Octyl-Cyanoacrylat enthält. Die Kunststoffampulle (11) wird vollständig von einem als äußeres Verpackungsmittel ausgebildeten Aluminiumbeutel (13) umgeben. Der Aluminiumbeutel (13) weist ein als Sterilisationsöffnung ausgebildetes Tyvek®-Fenster (14) auf. Nach Sterilisation der Verpackung (10) ist das Verpackungsvolumen (15) zwischen der Kunststoffampulle (11) und dem Aluminiumbeutel (13) steril. Das Tyvek®-Fenster (14) ist mit einer gasdichten Folie, beispielsweise mit einer Aluminiumklebefolie, verschließbar.
Fig. 3 zeigt eine Verpackung 20 mit einer als inneres Verpackungsmittel ausgebildeten Kunststoffampulle 21, die eine sterile und aushärtbare Klebstoffzusammensetzung 22 auf der Basis von n-Butyl-Cyanoacrylat (Histoacryl®) und Methoxypropyl-Cyanoacrylat enthält. Die Kunststoffampulle 21 ist von einem als äußeres Verpackungsmittel ausgebildeten Aluminiumbeutel 23 mit einer einseitigen als Sterilisationsöffnung ausgebildeten Öffnung 24 umgeben. Der Aluminiumbeutel 23 ist während der Sterilisation in einem als gasdurchlässiges und keimdichtes Behältnis ausgebildeten Tyvek®-Beutel 26 eingeschlossen. Nach Sterilisation wird die Verpackung 20 entlang einer gedachten Linie 27 durchgeschweißt, so dass die Kunststoffampulle 21 dampfdicht in dem Aluminiumbeutel 23 eingeschlossen ist, wobei das Verpackungsvolumen 25 steril ist. Der Tyvek®-Beutel 26 kann während des Verschließens oder nach dem Verschließen des Aluminiumbeutels 23 entfernt werden. Es ist auch möglich, insbesondere dann wenn der Tyvek^{®}-Beutel 26 beschriftet ist, diesen als zusätzlichen Schutz um die Verpackung 20 zu belassen.
Fig. 4 zeigt eine Verpackung 30 mit einer als inneres Verpackungsmittel ausgebildeten Kunststoffampulle 31, die eine sterile und aushärtbare Klebstoffzusammensetzung 32 auf der Basis von n-Butyl-Cyanoacrylat (Histoacryl^{®}) und Methoxypropyl-Cyanoacrylat enthält. Die Kunststoffampulle 31 ist von einem als äußeres Verpackungsmittel ausgebildeten Aluminiumbeutel 33 umgeben, der ein einseitig integriertes als Sterilisationsöffnung ausgebildetes Tyve^{®}-Fenster 34 aufweist. Nach Sterilisation wird die Verpackung 30 entlang einer gedachten Linie 37 unter Entfernung des Tyvek^{®}-Fensters 34 durchgeschweißt, so dass die Kunststoffampulle 31 dampfdicht im Aluminiumbeutel 33 eingeschlossen ist, wobei das Verpackungsvolumen 35 steril ist.

### Beispiel 1

Es wurden Klebstoffzusammensetzungen jeweils durch Sterilfiltration und aseptische Abfüllung in Kunststoffampullen überführt. Die Kunststoffampullen wurden anschließend versiegelt und jeweils in einen Beutel aus Tyvek^{®} verpackt. Ein Teil der Tyvek^{®}-beutel wurde daraufhin einer Gamma-Bestrahlung unterworfen. Anschließend wurde die Viskosität der Klebstoffzusammensetzungen jeweils nach bestimmten Zeiträumen untersucht. Die Ergebnisse sind in Tabelle 1 dargestellt.

**Tabelle 1: Vergleich der Viskosität von Klebstoffzusammensetzungen aus unbestrahlten und bestrahlten Tyvek^{®}-beuteln**

| Lagerzeit/-temperatur | Viskosität [mPas] ohne Bestrahlung | Viskosität [mPas] nach Bestrahlung |
|---|---|---|
| 4 Monate / 8 °C | 3,62 | 7,01 |
| 6 Monate / 8 °C | 3,71 | 15,36 |

Aus der Tabelle 1 geht deutlich hervor, dass die Sterilisierung der Beutel (Tyvek^{®}) mit Gamma-Bestrahlung zu einer Aushärtung der Klebstoffzusammensetzung führt.

### Beispiel 2

Es wurden Klebstoffzusammensetzungen jeweils durch Sterilfiltration und aseptische Abfüllung in Kunststoffampullen überführt. Die Kunststoffampullen wurden anschließend versiegelt und jeweils in einem Beutel aus Tyvek® verpackt. Ein Teil der Tyvek^{®}-beutel wurde daraufhin einer Elektronenstrahl-Bestrahlung unterworfen. Anschließend wurde die Haftkraft der Klebstoffzusammensetzungen jeweils nach bestimmten Zeiträumen untersucht. Die Ergebnisse sind in Tabelle 2 dargestellt.

**Tabelle 2: Vergleich der Haftkraft von Klebstoffzusammensetzungen aus unbestrahlten und bestrahlten Tyvek^{®}-beuteln**

| Lagerzeit/-temperatur | Haftkraft [N] ohne Bestrahlung | Haftkraft [N] nach Bestrahlung |
|---|---|---|
| 4 Monate / 8 °C | 58,4 N | 42,2 |
| 6 Monate / 8 °C | 58,9 N | 24,2 |
| 12 Monate / 8 °C | 69,2 N | 0 N |

Der Tabelle 2 ist deutlich zu entnehmen, dass die Haftkraft der durch Elektronenstrahl-Bestrahlung sterilisierten Tyvek^{®}-beutel rasch abnimmt und nach 12 Monaten den Wert Null erreicht. Dies bedeutet, dass die Klebstoffzusammensetzungen spätestens nach 12 Monaten keine Klebekraft mehr aufweisen. Entsprechende Ergebnisse wurden auch bei einer Ethylenoxid-Begasung bzw. einer Dampfsterilisation der Papierbeutel erhalten.

### Beispiel 3

Es wurden Klebstoffzusammensetzungen jeweils durch Sterilfiltration und aseptische Abfüllung in Kunststoffampullen überführt. Die Kunststoffampullen wurden anschließend versiegelt und jeweils in einem Pa-Beutel aus Tyvek^{®} verpackt. Ein Teil der Tyvek^{®}-beutel wurde daraufhin gemäß der vorliegenden Erfindung einer Wasserstoffperoxid-Plasma-Sterilisation unterworfen. Anschließend wurde die Viskosität der Klebstoffzusammensetzungen jeweils nach bestimmten Zeiträumen untersucht. Die Ergebnisse sind in Tabelle 3 dargestellt.

**Tabelle 3: Vergleich der Viskosität von Klebstoffzusammensetzungen aus nicht sterilisierten und durch Wasserstoffperoxid-Plasma sterilisierten Tyvek^{®}-beuteln**

| Lagerzeit/-temperatur | Viskosität [mPas] ohne Sterilisation | Viskosität [mPas] nach Sterilisation |
|---|---|---|
| 4 Monate / 8 °C | 4,02 | 3,64 |
| 6 Monate / 8 °C | 4,11 | 4,72 |

Aus der Tabelle 3 geht deutlich hervor, dass die Klebstoffzusammensetzungen aus durch Wasserstoffperoxid-Plasma sterilisierten Tyvek^{®}beuteln selbst nach einem Untersuchungszeitraum von 6 Monaten im Wesentlichen noch eine Viskosität, d.h. Aushärtbarkeit, aufwiesen, wie die entsprechenden Klebstoffzusammensetzungen aus den nicht sterilisierten Tyvek^{®}-beuteln.

Diese Ergebnisse lassen sich noch verbessern, wenn das äußere Verpackungsmittel gasdicht ist, und der Innenraum des äußeren Verpackungsmittels während oder nach der Sterilisation vollständig von Restfeuchtigkeit befreit wird, bevor das äußere Verpackungsmittel gasdicht verschlossen wird. Auf diese Weise kann eine Diffusion von Restfeuchtigkeit in die aus Kunststoff bestehenden Klebstoffampullen vermieden werden.

## Patentansprüche

1. Sterilisierte Verpackung (1; 10; 20; 30) mit einem inneren im Wesentlichen gasdichten und geschlossenen Verpackungsmittel (2; 11; 21; 31), das eine sterile und zu einem Kleber aushärtbare Klebstoffzusammensetzung (3; 12; 22; 32) enthält, und einem äußeren Verpackungsmittel (4; 13; 23; 34), das das innere Verpackungsmittel (2; 11; 21; 31) umgibt, wobei die Verpackung (1; 10; 20; 30) zwischen dem inneren Verpackungsmittel (2; 11; 21; 31) und dem äußeren Verpackungsmittel (4; 13; 23; 33) ein steriles Verpackungsvolumen (5; 15; 25; 35) aufweist.

2. Verpackung (1; 10; 20; 30) nach Anspruch 1, **dadurch gekennzeichnet, dass** das äußere Verpackungsmittel (4; 13; 23; 33) eine Sterilisationsöffnung (14; 24; 34), insbesondere eine gasdurchlässige und keimdichte Sterilisationsöffnung (14; 34), vorzugsweise in Form eines Wandungsteils, aufweist.

3. Verpackung (1; 10; 20; 30) nach Anspruch 1 oder 2, **dadurch gekennzeichnet, dass** das äußere Verpackungsmittel (4; 13; 23; 33) eine Sterilisationsöffnung (14; 24; 34) aufweist, die gasdicht verschlossen ist.

4. Verpackung (1; 10; 20; 30) nach einem der vorhergehenden Ansprüche, **dadurch gekennzeichnet, dass** das äußere Verpackungsmittel (4; 13; 23; 33) eine Sterilisationsöffnung (14; 34) aus einem Faservliesmaterial, insbesondere aus einem polymeren Faservliesmaterial, aufweist.

5. Verpackung (1; 10; 20; 30) nach Anspruch 4, **dadurch gekennzeichnet, dass** das Faservliesmaterial aus Polyethylen, vorzugsweise aus Hochdruckpolyethylen, gebildet ist.

6. Verpackung (1; 10; 20; 30) nach einem der vorhergehenden Ansprüche, **dadurch gekennzeichnet, dass** das innere Verpackungsmittel (2; 11; 21; 31) eine Ampulle, insbesondere auf der Basis von Kunststoffen, ist.

7. Verpackung (1; 10; 20; 30) nach einem der vorhergehenden Ansprüche, **dadurch gekennzeichnet, dass** das äußere Verpackungsmittel (4; 13; 23; 33) aus gasdichten Materialien, vorzugsweise aus Metallen oder Verbundmaterialien, gebildet ist und das Verpackungsvolumen (5; 15; 25; 35) des äußeren Verpackungsmittels (4; 13; 23; 33) vorzugsweise frei von Feuchtigkeit ist.

8. Verpackung (1; 10; 20; 30) nach einem der vorhergehenden Ansprüche, **dadurch gekennzeichnet, dass** ein SAL (sterile assurance level) zwischen dem inneren Verpackungsmittel (2; 11; 21; 31) und dem äußeren Verpackungsmittel (4; 13; 23; 33) < 10⁻³, vorzugsweise ≤ 10⁻⁶, ist.

9. Verpackung (1; 10; 20; 30) nach einem der vorhergehenden Ansprüche, **dadurch gekennzeichnet, dass** das innere Verpackungsmittel (2; 11; 21; 31) und/oder das äußere Verpackungsmittel (4; 13; 23; 33), vorzugsweise das innere Verpackungsmittel (2; 11; 21; 31) und das äußere Verpackungsmittel (4; 13; 23; 33), frei von Sterilisationsrückständen, insbesondere frei von Sterilisationsgasen, sind.

10. Verpackung (1; 10; 20; 30) nach einem der vorhergehenden Ansprüche, **dadurch gekennzeichnet, dass** die Klebstoffzusammensetzung (3; 12; 22; 32) in dem inneren Verpackungsmittel (2; 11; 21; 31) während eines Zeitraums von 2 bis 60 Monaten, insbesondere von 4 bis 36 Monaten, vorzugsweise von 6 bis 24 Monaten, nach Sterilisation der Verpackung (1; 10; 20; 30) eine Viskosität aufweist, die im Wesentlichen der Viskosität der Klebstoffzusammensetzung (3; 12; 22; 32) in dem inneren Verpackungsmittel (2; 11; 21; 31) vor Sterilisation der Verpackung (1; 10; 20; 30) entspricht.

11. Verpackung (1; 10; 20; 30) nach einem der vorhergehenden Ansprüche, **dadurch gekennzeichnet, dass** die Klebstoffzusammensetzung (3; 12; 22; 32) in dem inneren Verpackungsmittel (2; 11; 21; 31) während eines Zeitraums von 2 bis 60 Monaten, insbesondere von 4 bis 36 Monaten, vorzugsweise von 6 bis 24 Monaten, nach Sterilisation der Verpackung (1; 10; 20; 30) einen Wassergehalt aufweist, der im Wesentlichen dem Wassergehalt der Klebstoffzusammensetzung (3; 12; 22; 32) in dem inneren Verpackungsmittel (2; 11; 21; 31) vor Sterilisation der Verpackung (1; 10; 20; 30) entspricht.

12. Verpackung (1; 10; 20; 30) nach einem der vorhergehenden Ansprüche, **dadurch gekennzeichnet, dass** die Klebstoffzusammensetzung (3; 12; 22; 32) in dem inneren Verpackungsmittel (2; 11; 21; 31) während eines Zeitraums von mindestens 2 Monaten, insbesondere von mindestens 4 Monaten, vorzugsweise von mindestens 6 Monaten, nach Sterilisation der Verpackung (1; 10; 20; 30) eine Haftkraft aufweist, die im Wesentlichen der Haftkraft der Klebstoffzusammensetzung (3; 12; 22; 32) in dem inneren Verpackungsmittel (2; 11; 21; 31) vor Sterilisation der Verpackung (1; 10; 20; 30) entspricht.

13. Verfahren zur Sterilisation einer gasdurchlässigen und keimdichten Verpackung (1; 10; 20; 30) mit einem inneren im Wesentlichen gasdichten und geschlossenen Verpackungsmittel (2; 11; 21; 31), welches eine sterile und zu einem Kleber aushärtbare Klebstoffzusammensetzung (3; 12; 22; 32) enthält, und einem äußeren Verpackungsmittel (4; 13; 23; 33), welches das innere Verpackungsmittel (2; 11; 21; 31) umgibt, wobei die Verpackung (1; 10; 20; 30) einer Gassterilisation unter Erhalt der Aushärtbarkeit der Klebstoffzusammensetzung (3; 12; 22; 32) und unter Ausbildung eines sterilen Verpackungsvolumens (5; 15; 25; 35) zwischen dem inneren Verpackungsmittel (2; 11; 21; 31) und dem äußeren Verpackungsmittel (4; 13; 23; 33) unterworfen wird.

14. Verfahren nach Anspruch 13, **dadurch gekennzeichnet; dass** nach der Gassterilisation eine Sterilisationsöffnung (14; 24; 34), insbesondere eine gasdurchlässige und keimdichte Sterilisationsöffnung (14; 34), vorzugsweise in Form eines Wandungsteils, des äußeren Verpackungsmittels (4; 13; 23; 33) gasdicht verschlossen wird und der Verpackungsinhalt vorher vorzugsweise getrocknet wird.

15. Verfahren nach Anspruch 13 oder 14, **dadurch gekennzeichnet, dass** die Verpackung (1; 10; 20; 30) in Gegenwart von Radikalen, insbesondere Sauerstoffradikalen, vorzugsweise von Wasserstoffperoxid abgeleitet, sterilisiert wird.

16. Verfahren nach einem der Ansprüche 13 bis 15, **dadurch gekennzeichnet, dass** die Verpackung (1; 10; 20; 30) einer Plasma-Sterilisation, vorzugsweise einer Wasserstoffperoxid-Plasma-Sterilisation, unterworfen wird.

17. Verfahren nach einem der Ansprüche 13 bis 15, **dadurch gekennzeichnet, dass** die Verpackung (1; 10; 20; 30) mit Wasserstoffperoxid-Gas, vorzugsweise mit Wasserstoffperoxid-Dampf, sterilisiert wird.

18. Verfahren nach Anspruch 13 oder 14, **dadurch gekennzeichnet, dass** die Verpackung (1; 10; 20; 30) mit Formaldehyd-Gas, vorzugsweise mit Formaldehyd-Dampf, sterilisiert wird.

19. Verfahren nach einem der Ansprüche 13 bis 18, **dadurch gekennzeichnet, dass** die Verpackung (1; 10; 20; 30) bei Unterdruck, insbesondere bei einem Unterdruck zwischen 0,005 und 200 Torr, insbesondere zwischen 0,01 und 100 Torr, vorzugsweise zwischen 0,1 und 50 Torr, sterilisiert wird.

20. Verfahren nach einem der Ansprüche 13, 14, 17 oder 18, **dadurch gekennzeichnet, dass** die Verpackung (1; 10; 20; 30) bei Überdruck, insbesondere bei einem Überdruck zwischen 1 und 5 bar, vorzugsweise zwischen 1,05 und 5 bar, sterilisiert wird.

21. Verfahren nach einem der Ansprüche 13 bis 20, **dadurch gekennzeichnet, dass** die Verpackung (1; 10; 20; 30) bei einer Temperatur zwischen 20 und 75 °C, insbesondere bei einer Temperatur zwischen 25 und 60 °C, vorzugsweise bei einer Temperatur zwischen 30 und 50 °C, sterilisiert wird.

22. Verfahren nach einem der Ansprüche 13 bis 21, **dadurch gekennzeichnet, dass** die Verpackung (1; 10; 20; 30) während eines Zeitraums von 30 bis 240 min, insbesondere von 40 bis 120 min, vorzugsweise von ca. 60 min, sterilisiert wird.

23. Verfahren nach einem der Ansprüche 13 bis 22, **dadurch gekennzeichnet, dass** die Klebstoffzusammensetzung (3; 12; 22; 32) spätestens bei Überführung in das innere Verpackungsmittel (2; 11; 21; 31) steril gemacht wird.

24. Verfahren nach einem der Ansprüche 13 bis 23, **dadurch gekennzeichnet, dass**, die Klebstoffzusammensetzung (3; 12; 22; 32) durch Sterilfiltration und/oder aseptische Abfüllung, vorzugsweise durch Sterilfiltration und aseptische Abfüllung, steril gemacht wird.

25. Verpackung (1; 10; 20; 30), hergestellt nach einem Verfahren nach einem der Ansprüche 13 bis 24.

26. Verpackung (1; 10; 20; 30), herstellbar nach einem Verfahren nach einem der Ansprüche 13 bis 24.

27. Verwendung einer sterilisierten Verpackung (1; 10; 20; 30) nach einem der Ansprüche 1 bis 11 zur sterilen und trockenen Aufbewahrung des inneren Verpackungsmittels (2; 11; 21; 31) in der Verpackung (1; 10; 20; 30) und zur sterilen Entnahme des inneren Verpackungsmittels (2; 11; 21; 31) aus der Verpackung (1; 10; 20; 30), insbesondere zur sterilen Entnahme der aushärtbaren Klebstoffzusammensetzung (3; 12; 22; 32) aus dem inneren Verpackungsmittel (2; 11; 21; 31).
